# FASCICULE DE BREVET EUROPEEN

(11) **EP 0 768 884 B1**
(45) Date de publication et mention de la délivrance du brevet: **31.01.2001**
(21) Numéro de dépôt: 95923419.6
(22) Date de dépôt: 20.06.1995
(51) Int. Cl.: A61K 31/415

(54) **MEDICAMENTS A BASE D'UN MELANGE SYNERGETIQUE DE METRONIDAZOLE ET DE CLINDAMYCINE**
ARZNEIMITTEL AUF DER BASIS VON EINER SYNERGISTISCHEN MISCHUNG VON METRONIDAZOL UND CLINAMICIN
DRUGS CONTAINING A SYNERGISTIC MIXTURE OF METRONIDAZOLE AND CLINDAMYCIN

(30) Priorité: 06.07.1994 FR 9408350
(43) Date de publication de la demande: 23.04.1997
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: CZERNIELEWSKI, Janusz, F-06410 Biot (FR); ALLEC, Josiane "Les Vergers de Val Constance", F-06600 Antibes (FR); BOUCLIER, Martine "L'Ile Verte", F-06560 Valbonne (FR)
(74) Mandataire: Tezier Herman, Béatrice
(86) Numéro de dépôt international: FR9500819
(87) Numéro de publication internationale: WO9601117

(56) Documents cités:
- WO-A-88/06888
- US-A- 4 837 378
- US-A- 4 957 918
- J INFECT DIS,, VOL. 133, NO. 3. 1976 321-328, BUSCH D F et al 'ACTIVITY OF COMBINATIONS OF ANTI MICROBIAL AGENTS AGAINST BACTEROIDES-FRAGILIS'
- PEDIATR INFECT DIS J (UNITED STATES), MAR 1987, VOL. 6, NO. 3, PAGE(S) 332-5, Brook I 'Synergistic combinations of antimicrobial agents against anaerobic bacteria.'
- DRUGS UNDER EXPERIMENTAL AND CLINICAL RESEARCH, vol.VIII, no.3, 1982 pages 227 - 229 GARCIA-RODRIGUEZ, J. A. ET AL. 'Synergic effect of metaonidazole with other drusgs against bacteroides of the fragilis group'

## Description

La présente invention a trait, d'une manière générale, à l'utilisation d'une association de métronidazole et de clindamycine, comme principe actif, pour la fabrication de compositions pharmaceutiques, plus particulièrement dermatologiques, destinées à un traitement anti-inflammatoire curatif et/ou prophylactique, par voie topique.

Le métronidazole, ou 2-méthyl-5-nitroimidazole-1-éthanol, est un produit déjà connu en soi, largement utilisé pour le traitement par voie topique de la rosacée (acne rosacea), comme cela est par exemple décrit dans le brevet US 4 837 378. Toutefois, le ou les mécanismes d'action exacts par lesquelles le métronidazole traite cette affection restent, encore aujourd'hui, inconnus ou purement hypothétiques, certaines des explications avancées à cet égard n'étant en effet basées tout au plus que sur de simples modèles spéculatifs de type *in vitro.*

A l'inverse, il est maintenant bien établi que le métronidazole constitue un agent antimicrobien particulièrement actif, capable d'agir dans le traitement systémique de certaines infections anaérobies et parasitaires.

Ainsi, il n'existe, à la connaissance de la Demanderesse, aucune donnée publiée à ce jour concernant le potentiel anti-inflammatoire *in vivo* du métronidazole appliqué par voie topique.

Or, les travaux de la Demanderesse ont permis de mettre en évidence que le métronidazole présente une bonne activité anti-inflammatoire après application topique *in vivo.*

Par ailleurs, il a été trouvé une synergie, au niveau de cette activité anti-inflammatoire, absolument remarquable dans le cas particulier où le métronidazole ci-dessus est en outre associé (association nouvelle en soi, notamment à titre de médicament) à de la clindamycine. Ce dernier résultat est d'autant plus inattendu et surprenant que la clindamycine, qui est un antibiotique déjà connu et couramment utilisé dans le traitement de l'acnée par voie topique, ne présente en soi aucune, ou substantiellement aucune, activité anti-inflammatoire.

Toutes ces découvertes sont à la base de la présente invention.

D'autres caractéristiques, aspects, objets et avantages de l'invention apparaitront encore plus clairement à la lecture de la description qui va suivre, ainsi que des divers exemples concrets, mais nullement limitatifs, destinés à l'illustrer.

De préférence, la composition pharmaceutique est destinée à un usage topique.

Plus particulièrement, la composition pharmaceutique est donc une composition dermatologique.

Avantageusement, on utilise une association à base du mélange synergétique entre le métronidazole et la clindamycine pour la fabrication d'une composition pharmaceutique destinée à un traitement anti-inflammatoire.

La composition dermatologique est plus particulièrement destinée au traitement par voie topique de maladies ou affections cutanées présentant au moins une composante inflammatoire ou, à la fois, une composante inflammatoire et infectieuse.

Plus particulièrement, les maladies ou les affections de la peau correspondent à des inflammations cutanées accompagnant tout type de dermatoses telles que l'eczéma, le psoriasis, l'acné rosacé, l'acné vulgaire, les ulcères, les dermites séborrhéïques et les irritations induites par des agents chimiques, physiques ou mécaniques ou autres.

Dans ce qui suit, on entend par voie topique, toute technique d'administration d'un produit par application directe de ce dernier sur une partie superficielle (ou externe) du corps, telle que la peau, et par voie systémique, toute technique d'administration d'un produit par une voie autre que topique, par exemple orale et/ou parentérale.

L'administration des compositions selon l'invention peut donc être effectuée par voie entérale, parentérale, topique ou oculaire. Toutefois, de préférence, ces compositions sont conditionnées sous une forme convenant à une application par voie topique.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Par voie topique, les compositions pharmaceutiques à base de métronidazole ou de métronidazole et de clindamicyne, qui sont donc plus particulièrement destinées au traitement de la peau ou des muqueuses, peuvent se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée des actifs. Ces compositions par voie topique peuvent par ailleurs se présenter soit sous forme anhydre, soit sous une forme aqueuse, selon l'indication clinique. Des exemples de formulations à usage topique convenant particulièrement bien dans le cadre de la mise en oeuvre de la présente invention sont notamment donnés dans le brevet US-A- 4 837 378 précité, dont l'enseignement est, à cet égard, inclus à titre de référence dans la présente description.

Par voie oculaire, ce sont principalement des collyres.

Les compositions, de préférence à usage topique, selon l'invention contiennent du métronidazole à une concentration de préférence comprise entre 0,01 % et 5 % en poids par rapport au poids total de la composition, et de la clindamycine (lorsqu'elle est présente) à une concentration de préférence comprise entre 0,1 % et 10 % en poids par rapport au poids total de la composition.

Selon un mode particulier et préféré de réalisation de la présente invention, la teneur globale du mélange [métronidazole + clindamycine] n'excède pas 5 à 10 % du poids total des compositions médicamenteuses.

Les compositions médicamenteuses selon l'invention peuvent bien entendu en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétokonazole ou les polyméthylène-4,5 isothiazolidones-3; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à la composition pharmaceutique de manière telle que les propriétés avantageuses attachées intrinsèquement à la composition ne soient pas, ou substantiellement pas, altérées par l'addition envisagée.

On va maintenant donner, à titre nullement limitatif, plusieurs exemples destinés d'une part à démontrer les effets attachés à la présente invention, et, d'autre part, à illustrer diverses formulations concrètes conformes à l'invention.

### EXEMPLE 1

Cet exemple a pour but de mettre en évidence l'activité anti-inflammatoire topique *in vivo* du métronidazole et d'une association [métronidazole + clindamycine].

Le test utilisé pour évaluer cette activité est celui de l'oedème de l'oreille de souris induit par application topique d'acide arachidonique. Selon ce modèle, une application topique d'acide arachidonique sur l'oreille provoque une inflammation qui se caractérise par le développement rapide d'un oedème, ce dernier devenant maximal au bout d'une heure après l'application. La réponse oedémateuse est ensuite quantifiée par une mesure de l'épaisseur de l'oreille. Il est à noter que les anti-inflammatoires classiques non stéroïdiens tels que les inhibiteurs de cyclooxygénases ou de lipoxygénases (Indométhacine, Naproxen, Phénylbutazone,...) ainsi que les agents capables de bloquer la fuite plasmatique vasculaire (vaso constricteurs,...) sont de bons inhibiteurs dans ce modèle.

Le protocole opératoire exact est le suivant : les souris sont tout d'abord prétraitées avec le ou les produits actifs à évaluer, en procédant sur l'une de leur oreille à deux applications topiques par jour (2 x 25 µl ; une application le mâtin, l'autre le soir) d'une solution d'acétone contenant, à une concentration donnée, ce ou ces actifs ; ce pré-traitement est conduit pendant quatre jours consécutifs. Puis, le cinquième jour, les souris recoivent une dernière application (25 µl) de la solution contenant le ou les actifs à tester, et ceci deux heures avant l'application sur l'oreille ainsi pré-traitée de l'acide arachidonique destiné à générer l'oedème (25 µl d'une solution THF/Méthanol à 4% poids d'acide arachidonique). La réponse oedémateuse est alors quantifiée par une mesure de l'épaisseur de l'oreille 1 Heure et 2 Heures après l'application de la solution d'acide arachidonique. Les résultats sont ensuite exprimés en % d'inhibition (après 1 h et après 2 h) de l'oedème par rapport à l'oedème observé sur l'autre oreille qui n'avait été, quant à elle, pré-traitée (dans les mêmes conditions que ci-dessus) que par une solution d'acétone sans actif (oreille et oedème témoin ou de référence). Les résultats obtenus sont rassemblés dans le tableau ci-dessous.

| Traitement | Doses | % inhibition | |
|---|---|---|---|
| | | après 1 h | après 2 h |
| Métronidazole seul | 2 % | **20** | **36** |
| Clindamycine seule | 2 % | **(ni)** | **(ni)** |
| Métronidazole + Clindamycine | 2 % + 2 % | **46** | **63** |
| **(ni) : pas d'inhibition statistiquement significative** | | | |

Les résultats ci-dessus mettent clairement en évidence d'une part la bonne activité anti-inflammatoire du métronidazole seul dans le cas d'un traitement par voie topique, et, d'autre part, l'activité anti-inflammatoire remarquable qui est attachée à l'association [métronidazole + clindamycine] dans ce même traitement, alors que la clindamycine, seule, ne présente par elle-même aucune activité significative.

### EXEMPLE 2

On illustre ici un exemple concret de formulation conforme à l'invention se présentant sous la forme d'un gel à usage topique.
- Métronidazole 0,75 g
- Clindamycine phosphate 1,18 g
- Carbopol 980 (GOODRICH) 0,6 g
- Polyéthylène glycol 400 3 g
- Hydroxyde de sodium qs pH 5
- Conservateurs qs
- Eau déminéralisée qsp 100 g

### EXEMPLE 3

On illustre ici un exemple concret de formulation conforme à l'invention se présentant sous la forme d'une crème à usage topique.
- Métronidazole 0,75 g
- Sesquistéarate de méthyl glucose 1 g
- Alcool stéarylique 0,5 g
- Huile de vaseline fluide 6 g
- Polyéthylène glycol 400 2 g
- Sesquistéarate de méthyl glucose 5 g polyoxyéthyléné à 20 moles d'OE
- Carbopol 981 (GOODRICH) 0,4 g
- Glycérol 7 g
- Clindamycine phosphate 1,18 g
- Cyclométhicone 4 g
- Hydroxyde de sodium qs pH 5
- Conservateurs qs
- Eau déminéralisée qsp 100 g

## Revendications

1. Utilisation du métronidazole pour la fabrication d'une composition pharmaceutique destinée à un traitement anti-inflammatoire caractérisée en ce que le métronidazole est associé à la clindamycine.

2. Utilisation selon la revendication 1, caractérisée en ce que la composition est destinée à un usage topique.

3. Utilisation selon la revendication précédente, caractérisée par le fait que la clindamycine est présente à raison de 0,1 % à 10 % en poids par rapport au poids total de la composition.

4. Utilisation selon l'une quelconque des revendications 1 à 3, caractérisée par le fait que la teneur globale du mélange [métronidazole + clindamycine] n'excède pas 5 à 10 % du poids total de la composition.

5. Utilisation selon l'une quelconque des revendications précédentes, caractérisée par le fait que le métronidazole est présent à raison de 0,01 % à 5 % en poids par rapport au poids total de la composition pharmaceutique.

6. Utilisation selon l'une quelconque des revendications précédentes, caractérisée en ce que la composition pharmaceutique est une composition dermatologique.

7. Utilisation selon la revendication précédente, caractérisée en ce que la composition dermatologique est destinée au traitement de maladies ou affections cutanées présentant au moins une composante inflammatoire ou, à la fois, une composante inflammatoire et infectieuse.

8. Utilisation selon la revendication 7, caractérisée par le fait que lesdites maladies ou afffections cutanées consistent en des inflammations cutanées accompagnant tout type de dermatoses telles que l'eczéma, le psoriasis, l'acné rosacé, l'acné vulgaire, les ulcères, les dermites séborrhéïques et les irritations induites par des agents chimiques, physiques ou mécaniques.

## Patentansprüche

1. Verwendung von Metronidazol zur Herstellung einer pharmazeutischen Zusammensetzung, die zur entzündungshemmenden Behandlung vorgesehen ist, dadurch gekennzeichnet, daß das Metronidazol mit Clindamycin kombiniert ist.

2. Verwendung nach Anspruch 1, dadurch gekennzeichnet, daß die Zusammensetzung zur topischen Anwendung vorgesehen ist.

3. Verwendung nach dem vorhergehenden Anspruch, dadurch gekennzeichnet, daß das Clindamycin in einem Mengenanteil von 0,1 bis 10 Gew.-%, bezogen auf das Gesamtgewicht der Zusammensetzung, vorliegt.

4. Verwendung nach einem der Ansprüche 1 bis 3, dadurch gekennzeichnet, daß die Gesamtmenge des Gemisches [Metronidazol + Clindamycin] 5 bis 10 % des Gesamtgewichts der Zusammensetzung nicht übersteigt.

5. Verwendung nach einem der vorhergehenden Ansprüche, dadurch gekennzeichnet, daß das Metronidazol in einem Mengenanteil von 0,01 bis 5 Gew.-%, bezogen auf das Gesamtgewicht der pharmazeutischen Zusammensetzung, vorliegt.

6. Verwendung nach einem der vorhergehenden Ansprüche, dadurch gekennzeichnet, daß die pharmazeutische Zusammensetzung eine dermatologische Zusammensetzung ist.

7. Verwendung nach dem vorhergehenden Anspruch, dadurch gekennzeichnet, daß die dermatologische Zusammensetzung zur Behandlung von Krankheiten oder Erkrankungen der Haut vorgesehen ist, die mindestens eine entzündliche Komponente oder eine entzündliche Komponente und eine infektiöse Komponente gleichzeitig aufweisen.

8. Verwendung nach Anspruch 7, dadurch gekennzeichnet, daß die Krankheiten oder Erkrankungen der Haut Entzündungen der Haut darstellen, die mit beliebigen Dermatosen, wie Ekzemen, Psoriasis, Akne rosacea, Akne vulgaris, Geschwüren, seborrhoeischer Dermatitis, und Reizungen einhergehen, welche durch chemische, physikalische oder mechanische Mittel hervorgerufen werden.

## Claims

1. Use of metronidazole for the manufacture of a pharmaceutical composition intended for an antiinflammatory treatment, characterized in that the metronidazole is in combination with clindamycin.

2. Use according to Claim 1, characterized in that the composition is intended for topical use.

3. Use according to the preceding claim, characterized in that the clindamycin is present in a proportion of 0.1 % to 10 % by weight with respect to the total weight of the composition.

4. Use according to any one of Claims 1 to 3, characterized in that the overall content of the [metronidazole + clindamycin] mixture does not exceed 5 to 10 % of the total weight of the composition.

5. Use according to any one of the preceding claims, characterized in that the metronidazole is present in a proportion of 0.01 % to 5 % by weight with respect to the total weight of the pharmaceutical composition.

6. Use according to any one of the preceding claims, characterized in that the pharmaceutical composition is a dermatological composition.

7. Use according to the preceding claim, characterized in that the dermatological composition is intended for the treatment of skin diseases or complaints having at least one inflammatory component or, at the same time, one inflammatory and one infectious component.

8. Use according to Claim 7, characterized in that the said skin diseases or complaints consist of skin inflammations accompanying any type of dermatoses such as eczema, psoriasis, acne rosacea, acne vulgaris, ulcers, seborrhoeic dermatitis, and irritations induced by chemical, physical or mechanical agents.
